# FASCICULE DE BREVET EUROPEEN

(11) **EP 1 982 157 B1**
(45) Date de publication et mention de la délivrance du brevet: **19.10.2011**
(21) Numéro de dépôt: 07730829.4
(22) Date de dépôt: 18.01.2007
(51) Int. Cl.: G01N 1/18

(54) **APPAREIL POUR LE PRELEVEMENT AUTOMATIQUE D'ECHANTILLONS DANS UN ECOULEMENT CONTINU**
VORRICHTUNG ZUR AUTOMATISCHEN PROBENENTFERNUNG AUS EINEM DURCHFLUSS
APPARATUS FOR THE AUTOMATIC REMOVAL OF SAMPLES FROM A CONTINUOUS FLOW

(30) Priorité: 08.02.2006 FR 0601104
(43) Date de publication de la demande: 22.10.2008
(73) Titulaire: Institut de Recherche pour le Développement ( IRD), 75480 Paris Cedex 10 (FR)
(72) Inventeur: JANEAU, Jean-Louis, Scottsville, 3209 (ZA); SILVERA, Norbert, Bangkok 10903 (TH); BRICQUET, Jean-Pierre, F-34170 Castelnau-le-lez (FR)
(74) Mandataire: Grosset-Fournier, Chantal Catherine
(86) Numéro de dépôt international: PCT/FR2007/000092
(87) Numéro de publication internationale: WO 2007/090941

(56) Documents cités:
- WO-A-93/04355
- DE-A1- 2 415 011
- FR-A1- 2 260 793
- GB-A- 1 386 106
- GB-A- 2 294 544
- US-A- 4 415 011

## Description

La présente invention est relative à un appareil pour le prélèvement automatique d'échantillons d'eau, notamment pour assurer la collecte de tels échantillons dans un écoulement continu dont les caractéristiques varient dans le temps, en particulier dans une rivière ou similaire, pour permettre de mesurer divers paramètres sur ces échantillons, en faisant intervenir certaines données instantanées propres à cet écoulement tels que son débit et sa vitesse.

Plus spécialement, l'invention concerne un appareil apte à réaliser une succession de prélèvements dans l'écoulement, séquencés en fonction du temps et/ou de la hauteur d'eau dans celui-ci, pour mesurer notamment sur chaque échantillon correspondant à un tel prélèvement, la quantité de particules en suspension et sa turbidité par unité de volume, en fonction de la vitesse et du débit de l'écoulement, également de la variation de niveau de celui-ci et de la quantité totale d'eau par unité de volume, variant par exemple en cas de pluie ou d'autres précipitations.

Des dispositifs de prélévement représentatifs de l'état de la technique sont décrits dans les documents GB 2294544, WO 93/04335, GB 1386106, DE 2415011, US 4415011 er FR 226793.

La mesure de la quantité d'eau, de son niveau,de son débit ainsi que du volume et de la concentration des sédiments dans un écoulement continu, est en général effectuée par des moyens optiques qui sont coûteux et peu adaptés.

L'invention propose un appareil propre à réaliser un échantillonnage à intervalles de temps donnés ou en fonction de la variation de la hauteur d'eau dans l'écoulement, en vue de mesurer de manière plus simple et moins onéreuse sur chaque échantillon, les paramètres recherchés, indépendamment d'autres mesures qui peuvent être effectuées simultanément ou séparément par le même dispositif d'évaluation.

A cet effet, l'appareil considéré, comportant un bâti fixe comprenant un plateau horizontal de support d'une pluralité de récipients propres à recevoir chacun, par son orifice de remplissage supérieur ouvert, un échantillon d'eau déterminé, ces récipients étant répartis selon une disposition circulaire sur ce plateau autour d'un axe vertical central selon lequel pivote une couronne d'entraînement parallèle au plateau, cette couronne étant solidaire d'une conduite d'amenée d'eau s'étendant perpendiculairement au plateau et raccordée à une extrémité, à travers un joint tournant, à une pompe d'aspiration de l'eau à partir de l'écoulement et dont l'autre extrémité est réunie à un bras distributeur horizontal, s'étendant radialement au-dessus des récipients disposés sur le plateau de support, ce bras distributeur délivrant successivement à chacun de ces récipients par son extrémité opposée à l'axe, un échantillon d'eau suite à la rotation de ce bras sous l'effet de la couronne d'entraînement, se caractérise en ce que le plateau horizontal de support comporte, au-dessus des orifices ouverts des récipients, un disque de répartition, parallèle au plateau et présentant des passages de distribution en nombre pair, égal au double de celui des récipients, ces passages étant pour moitié d'entre eux disposés chacun en regard d'un orifice ouvert d'un de ces récipients et séparés deux à deux par un passage intermédiaire analogue d'évacuation tel que, entre deux échantillons recueillis dans deux récipients successifs, l'eau délivrée par le bras distributeur soit évacuée par un passage intermédiaire permettant de réaliser une purge de ce bras et de la conduite d'amenée après remplissage d'un récipient quelconque par un échantillon et avant remplissage du récipient suivant, consécutivement à la rotation du bras.

Selon une caractéristique complémentaire de l'appareil considéré, la couronne d'entraînement du conduit d'amenée d'eau comporte une denture périphérique inclinée, en prise avec une vis hélicoïdale de commande, entraînée en rotation continue par un moteur électrique asynchrone.

En variante, la couronne d'entraînement comporte une denture périphérique droite, en prise avec un pignon.

Selon une autre caractéristique, chacun des passages de distribution prévus dans le disque de répartition comporte un court élément de tuyauterie souple, propre à guider l'échantillon d'eau vers l'orifice de remplissage ouvert de chaque récipient ou à assurer l'évacuation de la purge du conduit d'amenée et du bras distributeur, entre le remplissage de deux récipients successifs.

Avantageusement, les récipients sont disposés sur le plateau de support horizontal avec une répartition en quinconce sur la périphérie de ce plateau, sous les passages de distribution prévus sur le disque de répartition.

Dans un mode de réalisation préféré de l'appareil de prélèvement selon l'invention, le plateau de support horizontal comporte un bord externe relevé, pour le positionnement et l'immobilisation des récipients disposés sous le disque de répartition.

Dans le même mode de réalisation, le plateau de support comporte une surface grillagée ou perforée sur laquelle reposent les récipients, notamment pour permettre l'écoulement de l'eau de purge délivrée par le bras distributeur entre les remplissages de deux récipients successifs.

Selon encore une autre caractéristique, le bâti fixe peut être associé à un capot extérieur de protection amovible, dont le retrait permet le libre accès au disque de répartition.

D'autres caractéristiques d'un appareil de prélèvement automatique d'échantillons d'eau dans un écoulement continu, apparaîtront encore à travers la description qui suit d'un exemple de réalisation, donné à titre indicatif et non limitatif, en référence aux dessins annexés sur lesquels :
- La Figure 1 est une vue en perspective éclatée de l'appareil considéré ;
- La Figure 2 est une vue également en perspective du même appareil, mais dans lequel ses diverses pièces sont représentées assemblées ;
- La Figure 3 est une vue schématique d'une variante relative à un détail entrant dans la réalisation de l'appareil.

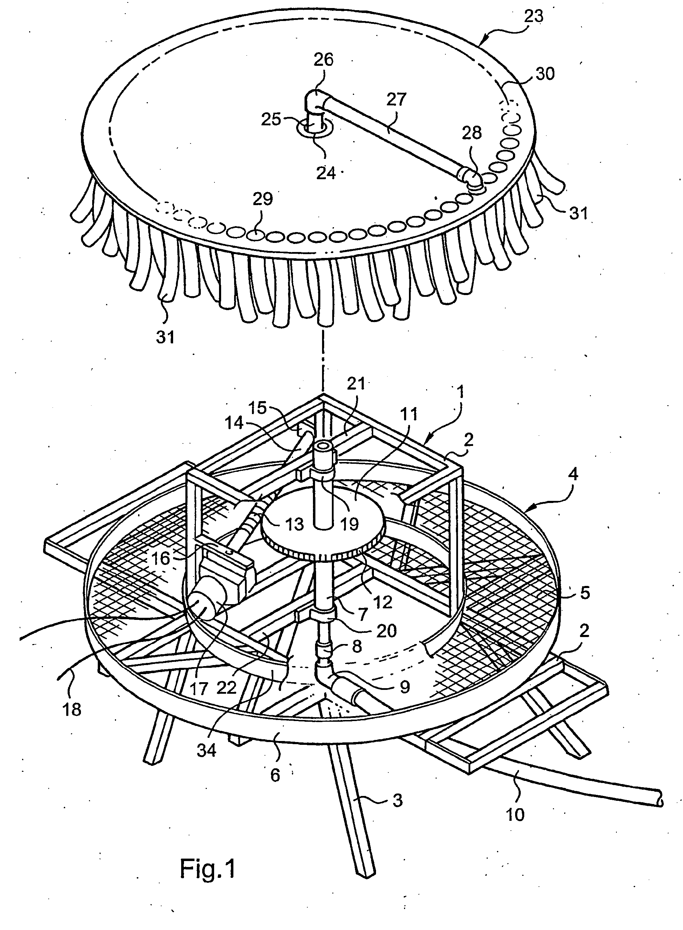

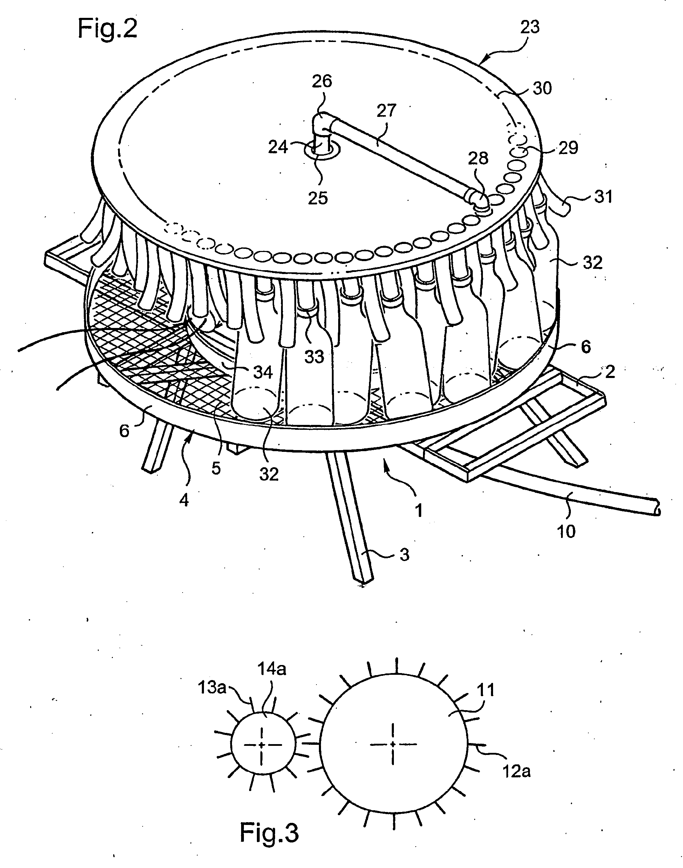

Sur les Figures 1 et 2, la référence 1 désigne le bâti fixe de l'appareil de prélèvement considéré, constitué par l'assemblage de profilés métallique 2, convenablement soudés ou autrement reliés entre eux, ce bâti comportant également des pieds d'appui au sol 3 permettant de positionner l'appareil en un endroit quelconque approprié.

Les pieds 3 sont solidarisés du bâti 1 et de préférence sont articulés sur celui-ci afin de faciliter, une fois le repliement de ses pieds effectué, le déplacement et le transport de ce bâti d'un endroit à un autre, selon le lieu où les prélèvements sont à réaliser.

Le bâti 1 comporte par ailleurs un plateau de support horizontal 4, prévu à sa partie inférieure, au-dessus des pieds d'appui 3, ce plateau présentant une surface grillagée 5 ou largement perforée pour les raisons exposées ci-après. La périphérie de ce plateau est munie d'un bord externe 6, circulaire et légèrement relevé pour s'étendre vers le haut à partir de la surface grillagée 5.

Au centre du plateau de support horizontal 4, est monté, à l'intérieur du bâti 1, un conduit d'amenée 7 qui s'étend verticalement perpendiculairement au plan du plateau et qui est relié par un dispositif à joint tournant 8, du genre presse-étoupe ou analogue, à un raccord 9, assurant la liaison de ce conduit avec l'extrémité d'une tuyauterie 10 dont l'autre extrémité plonge dans un écoulement d'eau (non représenté), situé à proximité et à partir duquel l'appareil permet d'effectuer les prélèvements successifs souhaités.

Notamment, la tuyauterie 10 est munie d'une pompe d'aspiration classique (non représentée) qui permet d'effectuer ces prélèvements dans l'écoulement d'eau, soit à intervalles de temps déterminés, de préférence identiques de l'un à l'autre, soit en fonction de la variation du niveau de l'eau dans l'écoulement, en particulier chaque fois que la hauteur d'eau augmente d'une valeur déterminée.

Dans sa partie sensiblement médiane, le conduit vertical 7 d'amenée d'eau est solidaire d'une couronne 11, s'étendant horizontalement à l'intérieur du bâti 1, parallèlement au plateau de support 4, cette couronne comportant à sa périphérie une denture 12 qui, dans l'exemple illustré sur la Figure 1, est inclinée de manière à coopérer avec la partie filetée 13 d'une vis sans fin 14, en prise avec elle.

Bien entendu, une telle disposition n'a qu'un caractère indicatif, la vis sans fin pouvant être substituée par un pignon 14a dont les dents 13a coopèrent avec une denture homologue 12, cette fois droite, prévue à la périphérie de la couronne d'entraînement 11, comme schématiquement représenté sur la vue de détail de la Figure 3.

La vis 14 tourillonne dans des paliers de support 15 et 16 fixés sur deux profilés 2 du bâti 1 et est entraînée en rotation continue sur elle-même au moyen d'un moteur asynchrone 17 dont les câbles d'alimentation 18 sont réunis, à distance convenable de l'appareil, à une source de tension électrique appropriée.

Le conduit d'amenée 7 tourne ainsi à l'intérieur du bâti 1 en étant maintenu axialement dans celui-ci par des goussets 19 et 20, convenablement soudés sur des traverses, respectivement 21 et 22, du bâti.

La Figure 1 illustre en vue éclatée un disque de répartition 23, s'étendant horizontalement, la Figure 2 montrant le positionnement de ce disque sur le bâti 1 une fois l'assemblage des diverses parties de l'appareil effectué.

Le disque de répartition 23 présente un profil circulaire dont le diamètre est sensiblement égal à celui du bord relevé 6 du plateau de support 4 et comporte une ouverture centrale 24 par laquelle l'extrémité supérieure 25 du conduit d'amenée 7 dépasse légèrement au-dessus du disque lorsque ce dernier est mis en place.

Cette extrémité 25 du conduit 7 est raccordée par un élément coudé 26 à une extrémité d'un bras distributeur horizontal 27 qui s'étend radialement au-dessus du disque de répartition 23, parallèlement à celui-ci, ce bras comportant à son extrémité opposée au conduit, un godet 28 par lequel l'eau prélevée, refoulée dans le conduit 7 et au-delà de celui-ci dans le bras 27 par la pompe d'aspiration, est susceptible d'être délivrée pour s'écouler à travers le disque 23 par un quelconque d'une pluralité de passages de distribution 29.

Ces passages 29 sont répartis selon un cercle 30 sur la périphérie du disque 23 et sont en nombre pair, chacun d'eaux étant disposé à la même distance du centre du disque, de manière à pouvoir être un à un successivement desservi par le godet 28 en bout du bras distributeur 27.

Chaque passage 29 est par ailleurs associé à un court élément de tuyauterie 31, qui lui est raccordé de façon étanche et s'étend sur une distance convenable sous la surface du disque de répartition 23.

Comme représenté sur la Figure 2, le plateau 4 solidaire du bâti fixe 1, supporte sur sa surface grillagée 5, un ensemble de récipients 32, du genre bouteille ou autre, de préférence en matière plastique, ces récipients 32 comportant chacun un orifice de remplissage supérieur 33 dont le diamètre est tel que puisse librement s'y engager l'extrémité d'un élément de tuyauterie 31 porté par le disque 23, de manière à ce qu'un échantillon d'eau prélevé dans l'écoulement et délivré par le bras distributeur 27 à travers le passage 29 du disque associé à cet élément, se recueille dans le récipient qui reçoit ce dernier.

Conformément à l'invention, le nombre des récipients 32 disposés sur la surface grillagée 5 du plateau de support 4 est égal à la moitié de celui des passages 29 prévus dans le disque de répartition 23, les passages associés à deux récipients successifs étant séparés par un passage homologue dont l'élément de tuyauterie 31 est libre, l'eau délivrée par cet élément, lorsque le bras distributeur 27 est situé au-dessus de ce passage, se répandant sur le sol à travers les mailles de la surface grillagée 5, en dehors des récipients 32.

Cette disposition présente l'avantage d'autoriser la purge de la tuyauterie 10, du conduit d'amenée 7 et du bras distributeur 27 après chaque prélèvement admis dans un récipient 32, avant que le prélèvement suivant ne soit recueilli dans un autre récipient situé sur le plateau de support 4 après le précédent.

Avantageusement, les récipients 32 ainsi répartis sur le plateau de support 4 sont disposés en quinconce de l'un à l'autre en étant maintenus vers l'extérieur du plateau par le bord relevé 6 qui s'étend à la périphérie de ce dernier. Un bord analogue 34, parallèle au bord 6, peut également être prévu sur' le plateau, plus à l'intérieur du bâti 1 afin de mieux maintenir en position l'ensemble des récipients.

De préférence également, l'appareil peut comporter un capot amovible (non représenté pour ne pas surcharger le dessin), ce capot se disposant au-dessus du disque de répartition 23 et du bras distributeur 27 et comportant un retour circulaire dirigé vers le bas pour entourer extérieurement l'ensemble des récipients 32 placés sur le plateau de support 4.

On réalise ainsi un appareil de prélèvement simple et peu coûteux, très robuste et par conséquent susceptible d'être utilisé dans des conditions climatiques parfois difficiles.

Les récipients contenant les échantillons prélevés, recueillis à partir du bras distributeur, peuvent ensuite être aisément repris un à un sur le plateau de support en vue de procéder, sur l'échantillon correspondant, aux mesures prévues à l'aide d'un appareil séparé qui, avantageusement, est conçu pour délivrer, outre une évaluation des paramètres propres à l'échantillon, comme notamment la quantité de sédiments qu'il comporte ou une estimation de sa turbidité, d'autres informations concernant la vitesse et le débit de l'écoulement d'où proviennent les échantillons ou la hauteur d'eau instantanée de celui-ci.

Bien entendu, il va de soi que l'invention ne se limite pas à l'exemple de réalisation plus spécialement décrit ci-dessus et représenté sur les dessins annexés ; elle en embrasse au contraire toutes les variantes entrant dans le champ des revendications ci-après.

## Revendications

1. - Appareil pour le prélèvement automatique d'échantillons d'eau dans un écoulement continu, comportant un bâti fixe (1) comprenant un plateau horizontal (4) de support d'une pluralité de récipients (32) propres à recevoir chacun, par son orifice de remplissage supérieur (33) ouvert, un échantillon d'eau déterminé, ces récipients étant répartis selon une disposition circulaire sur ce plateau autour d'un axe vertical central selon lequel pivote une couronne d'entraînement (11) parallèle au plateau, cette couronne étant solidaire d'une conduite d'amenée d'eau (7) s'étendant perpendiculairement au plateau et raccordée à une extrémité, à travers un joint tournant (8), à une pompe d'aspiration de l'eau à partir de l'écoulement et dont l'autre extrémité (25) est réunie à un bras distributeur horizontal (27), s'étendant radialement au-dessus des récipients disposés sur le plateau de support, ce bras distributeur délivrant successivement à chacun de ces récipients par son extrémité (28) opposée à l'axe, un échantillon d'eau suite à la rotation de ce bras sous l'effet de la couronne d'entraînement, **caractérisé en ce que** le plateau horizontal de support (4) comporte, au-dessus des orifices ouverts des récipients, un disque de répartition (23), parallèle au plateau et présentant des passages de distribution (29) en nombre pair, égal au double de celui des récipients (32), ces passages étant pour moitié d'entre eux disposés chacun en regard d'un orifice ouvert (33) d'un de ces récipients et séparés deux à deux par un passage intermédiaire analogue d'évacuation tel que, entre deux échantillons recueillis dans deux récipients successifs, l'eau délivrée par le bras distributeur (27) soit. évacuée par un passage intermédiaire permettant de réaliser une purge de ce bras et de la conduite d'amenée (7) après remplissage d'un récipient quelconque par un échantillon et avant remplissage du récipient suivant, consécutivement à la rotation du bras.

2. - Appareil- selon la revendication 1, **caractérisé en ce que** la couronne d'entraînement (11) du conduit (7) d'amenée d'eau comporte une denture périphérique inclinée (12), en prise avec une vis hélicoïdale (13) de commande, entraînée en rotation continue par un moteur électrique asynchrone.

3. - Appareil selon la revendication 1, **caractérisé en ce que** la couronne d'entraînement (11) comporte une denture périphérique droite (13a), en prise avec un pignon (14a).

4. - Appareil selon l'une quelconque des revendications 1 à 3, **caractérisé en ce que** chacun des passages de distribution (29) prévus dans le disque de répartition (23) comporte un court élément de tuyauterie souple (31), propre à guider l'échantillon d'eau vers l'orifice de remplissage ouvert (33) de chaque récipient (32) ou à assurer l'évacuation de la purge du conduit d'amenée (7) et du bras distributeur (27), entre le remplissage de deux récipients successifs.

5. - Appareil selon l'une quelconque des revendications 1 à 4, **caractérisé en ce que** les récipients (32) sont disposés sur le plateau de support horizontal (4) avec une répartition en quinconce sur la périphérie de ce plateau, sous les passages de distribution (29) sur le disque de répartition (23).

6. - Appareil selon l'une quelconque- des revendications 1 à 5, **caractérisé en ce que** le plateau de support horizontal (4) comporte un bord externe relevé (6), pour le positionnement et l'immobilisation des récipients disposés sous le disque de répartition (23).

7. - Appareil selon l'une quelconque des revendications 1 à 6, **caractérisé en ce que** le plateau de support (4) comporte une surface grillagée ou perforée (5) sur laquelle reposent les récipients (32), pour permettre l'écoulement de l'eau de purge délivrée par le bras distributeur (27) entre les remplissages de deux récipients successifs.

8. - Appareil selon l'une quelconque des revendications 1 à 7, **caractérisé en ce que** le bâti fixe (1) est associé à un capot extérieur de protection amovible, dont le retrait permet le libre accès au disque de répartition (23).

## Claims

1. - Apparatus for the automatic taking of samples of water from a continuous flow, comprising a fixed frame (1) comprising a horizontal platform (4) for the support of a plurality of containers (32) each designed to receive, by its upper open filling aperture (33), a determined sample of water, these containers being distributed according to a circular arrangement on this platform about a central vertical shaft along which pivots a driving ring gear (11) parallel to the platform, this ring gear being mounted on a water supply pipe (7) extending perpendicularly to the platform and connected at one end, through a rotating joint (8), to a pump for suction of the water from the continuous flow and the other end of which (25) is joined to a horizontal dispensing arm (21), extending radially above the containers arranged on the support platform, this dispensing arm successively delivering a water sample to each of these containers, by its end (28) opposite the shaft, following the rotation of this arm under the effect of the driving ring gear, **characterized in that** the supporting horizontal platform (4) comprises, above the open apertures of the containers, a distribution disc (23), parallel to the platform and having an even number of dispensing openings (29), equal to twice that of the containers (32), half of these openings each being arranged in front of an open aperture (33) of one of these containers and separated from each other by a similar intermediate discharge opening such that, between two samples collected in two successive containers, the water delivered by the dispensing arm (27) is discharged via an intermediate opening making it possible to carry out a flushing of this arm and of the supply pipe (7) after filling of any container with a sample and before filling of the next container, following the rotation of the arm

2. - Apparatus according to claim 1, **characterized in that** the driving ring gear (11) of the water supply pipe (7) comprises a set of inclined peripheral gear teeth (12), in engagement with a worm gear (13), driven in continuous rotation by an asynchronous electric motor.

3. - The apparatus according to claim 1, **characterized in that** the driving ring gear (11) comprises a set of upright peripheral gear teeth (13a), in engagement with a pinion (14a),

4. - Apparatus according to any one of claims 1 to 3, **characterized in that** each of the dispensing openings (29) provided in the distribution disc (23) comprises a short flexible tubing element (31), designed to guide the sample of water towards the open filling aperture (33) of each container (32) or to ensure the discharge of the flushing of the supply pipe (7) and the dispensing arm (27), between the filling of two successive containers.

5. - Apparatus according to any one of claims 1 to 4, **characterized in that** the containers (32) are arranged on the horizontal support platform (4) with staggered distribution over the periphery of this platform, under the dispensing openings (29) on the distribution disc (23).

6. - Apparatus according to any one of claims 1 to 5, **characterized in that** the horizontal support platform (4) comprises a raised outer edge (6), for the positioning and immobilization of the containers arranged under the distribution disc (23).

7. - Apparatus according to any one of claims 1 to 6, **characterized in that** the support platform (4) comprises a meshed or perforated surface (5) on which the containers (32) rest, to allow the flow of the flushing water delivered by the dispensing arm (27) between the fillings of two successive containers.

8. - Apparatus according to any one of claims 1 to 7, **characterized in that** the fixed frame (1) is associated with a removable outer protective cover, removal thereof allowing free access to the distribution disc (23).

## Patentansprüche

1. Vorrichtung zur automatischen Entnahme von Wasserproben aus einer kontinuierlichen Strömung, wobei die Vorrichtung ein feststehendes Gestell (1) umfasst, das eine horizontale Platte (4) zum Tragen mehrerer sauberer Behälter (32) umfasst, die dazu geeignet sind, jeweils in ihrer oberen offenen Einfüllöffnung (33) eine bestimmte Wasserprobe aufzunehmen, wobei diese Behälter entsprechend einer kreisförmigen Anordnung auf dieser Platte um eine zentrale vertikale Achse verteilt sind, gemäß der ein Antriebskranz (11) parallel zu der Platte gedreht wird, wobei dieser Kranz fest mit einer Wasserzulaufleitung (7) verbunden ist, die sich senkrecht zu der Platte erstreckt und an einem Ende mittels einer Drehkupplung (8) mit einer Pumpe zum Ansaugen von Wasser aus der Strömung angeschlossen ist und deren anderes Ende (25) mit einem horizontalen Verteilerarm (27) zusammengeführt ist, der sich radial über den auf der Trageplatte angeordneten Behälter erstreckt, wobei dieser Verteilerarm über sein Ende (28), das der Achse gegenüber liegt, nacheinander in jeden dieser Behälter eine Wasserprobe bezüglich der Drehung dieses Arms unter Einwirkung des Antriebskranzes abgibt, **dadurch gekennzeichnet, dass** die horizontale Trageplatte (4) über den offenen Öffnungen der Behälter eine Verteilungsscheibe (23) umfasst, die parallel zu der Platte ist und eine gerade Anzahl von Verteilungsdurchlässen (29) aufweist, wobei die Anzahl dem Doppelten der Anzahl der Behälter (32) entspricht, wobei die Hälfte dieser Durchlässe jeweils in Bezug auf eine offene Öffnung (33) eines dieser Behälter angeordnet ist und dies Durchlässe paarweise durch einen entsprechenden Zwischendurchlass zum Ablassen getrennt sind, so dass zwischen zwei Proben, die in zwei aufeinander folgenden Behältern aufgefangen wurden, das von dem Verteilerarm (27) abgegebene Wasser durch einen Zwischendurchlauf abgelassen wird, um eine Spülung dieses Arms und der Zulaufleitung (7) nach dem Befüllen eines beliebigen Behälters mit einer Probe und vor dem Befüllen des folgenden Behälters, der Drehung des Arms folgend, durchzuführen.

2. Vorrichtung nach Anspruch 1, **dadurch gekennzeichnet, dass** der Antriebskranz (11) der wasserzulaufleitung (7) eine geneigte Umfangsverzahnung (12) bezüglich einer Verteilersteuerschraube (13) umfasst, die in einer kontinuierlichen Drehung von einem Asynchronelektromotor angetrieben wird.

3. Vorrichtung nach Anspruch 1, **dadurch gekennzeichnet, dass** der Antriebskranz (11) eine aufrecht stehende Umfangsverzahnung (13a) bezüglich eines Zahnrads (14a) umfasst.

4. Vorrichtung nach einem der Ansprüche 1 bis 3, **dadurch gekennzeichnet, dass** jeder der Verteilungsdurchlässe (29), die in der Verteilungsscheibe (23) vorgesehen sind, ein kurzes Schlauchleitungselement (31) umfasst, das dazu geeignet ist, die Wasserprobe zur offenen Einfüllöffnung (33) jedes Behälters (32) zu leiten oder das Ablassen der Spülung der Zulaufleitung (7) und des Verteilerarms (27) zwischen der Befüllung von zwei aufeinander folgenden Behältern sicherzustellen.

5. Vorrichtung nach einem der Ansprüche 1 bis 4, **dadurch gekennzeichnet, dass** die Behälter (32) auf der horizontalen Trageplatte (4) in einer versetzten Verteilung auf dem Umfang dieser Platte unter den Verteilungsdurchlässen (29) auf der Verteilungsscheibe (23) angeordnet sind.

6. Vorrichtung nach einem der Ansprüche 1 bis 5, **dadurch gekennzeichnet, dass** cie horizontale Trageplatte (4) eine erhöhte äußere Kante (6) zur Positionierung und Immobilisierung von unter der Verteilungsscheibe (23) angeordneten Behältern umfasst.

7. Vorrichtung nach einem der Ansprüche 1 bis 6, **dadurch gekennzeichnet, dass** die Trageplatte (4) eine vergitterte oder perforierte Oberfläche (5) umfasst, auf der die Behälter (32) ruhen, um die Strömung von Spülwasser, das von dem Verteilerarm (27) abgegeben wurde, zwischen den Befüllungen von zwei aufeinander folgenden Behältern zu ermöglichen.

8. Vorrichtung nach einem der Ansprüche 1 bis 7, **dadurch gekennzeichnet, dass** das feststehende Gestell (1) mit einer abnehmbaren äußeren Schutzhaube verbunden ist, deren Entfernen freien Zugriff auf die Verteilungsscheibe (23) ermöglicht.
